# EUROPÄISCHE PATENTSCHRIFT

(11) **EP 0 938 889 B1**
(45) Veröffentlichungstag und Bekanntmachung des Hinweises auf die Patenterteilung: **22.11.2006**
(21) Anmeldenummer: 99103605.4
(22) Anmeldetag: 24.02.1999
(51) Int. Cl.: A61Q 5/00, A61K 8/81, A61K 8/87, A61K 8/90, D06C 29/00, D21H 19/62

(54) **Verwendung von Polyurethan in Haarfestigungsmitteln oder in Bindemitteln der pharmazeutischen Industrie sowie Beschichtungsmittel für die Textil- und Papierindustrie**
Use of polyurethane in hair fixative preparations as well as in binding compositions for the pharmaceutical industriy and in coating agents of the textile and paper industries
Utilisation du polyuréthane dans les fixateurs des cheveux ou dans les liants de l'industrie pharmaceutique et dans les agents de revêtement des industries textile et du papier

(30) Priorität: 25.02.1998 DE 19807908
(43) Veröffentlichungstag der Anmeldung: 01.09.1999
(73) Patentinhaber: BASF Aktiengesellschaft, 67056 Ludwigshafen (DE)
(72) Erfinder: Son, Nguyen Kim, 69502 Hemsbach (DE); Sanner, Axel, 67227 Frankenthal (DE); Dausch, Wilma, 67117 Limburgerhof (DE); Schehlmann, Volker, 67105 Schifferstadt (DE)
(74) Vertreter: Kinzebach, Werner

(56) Entgegenhaltungen:
- EP-A- 0 389 386
- WO-A-97/17386
- US-A- 4 719 132
- DATABASE CAPLUS [Online] CHEMICAL ABSTRACTS SERVICE, COLUMBUS, OHIO, US; retrieved from STN Database accession no. 126:119014/DN XP002249175 & JP 08 311774 A (DAI ICHI KOGYO SEIYAKU CO.) 26. November 1996 (1996-11-26)
- DATABASE CAPLUS [Online] CHEMICAL ABSTRACTS SERVICE, COLUMBUS, OHIO, US; retrieved from STN Database accession no. 130:97003/DN XP002249176 & JP 10 330683 A (DAINIPPON INK AND CHEMICALS) 15. Dezember 1998 (1998-12-15)

## Beschreibung

Die vorliegende Erfindung betrifft ein kosmetisches Mittel, welches wenigstens ein wasserlösliches oder wasserdispergierbares Polyurethan auf Basis von Polytetrahydrofuranen, Polysiloxanen und Mischungen davon sowie mindestens ein Polyesterdiol enthält.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15°C) besitzen.

Bei der Formulierung von Haarfestigern ist außerdem zu berücksichtigen, dass aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Ein weiterer aktueller Anspruch an Haarbehandlungsmittel ist es, dem Haar ein natürliches Aussehen und Glanz zu verleihen, z. B. auch dann, wenn es sich um von Natur aus besonders kräftiges und/ oder dunkles Haar handelt.

Die DE-A-39 01 325 und die DE-A-43 14 305 beschreiben Haarfestigungsmittel, die als Filmbildner Copolymerisate auf der Basis von A) t.-Butylacrylat und/oder t.-Butylmethacrylat, B) Acrylsäure und/oder Methacrylsäure und C) eines weiteren radikalisch copolymerisierbaren Monomeren enthalten, wobei die Carboxylgruppen der Copolymerisate teilweise oder vollständig neutralisiert sind. Haarfestiger auf Basis solcher Copolymerisate, die Carboxylgruppen enthaltende Monomere umfassen, weisen zwar im Allgemeinen befriedigende Eigenschaften in Bezug auf die erzielte Festigung, die Elastizität sowie die Auswaschbarkeit vom Haar auf, der Griff, der mit diesen Polymeren gefestigten Frisuren wird jedoch als unangenehm stumpf und unnatürlich empfunden. Der Versuch, diesen Effekt durch Zugabe üblicher Weichmacher zu vermindern, führt im Allgemeinen nicht zu befriedigenden Ergebnissen. So wird eine Verbesserung des Griffes im Allgemeinen mit einer reduzierten Festigungswirkung erkauft.

Es ist bekannt, Polysiloxane, wie z. B. Polydimethylsiloxan, und Polysiloxanderivate in Haarpflegemitteln einzusetzen.

Die EP-A-017 122 beschreibt die Verwendung von Polysiloxan-Ammoniumderivaten in Haarwasch- und Haarbehandlungsmitteln zur Verbesserung der Kämmbarkeit, Weichheit und Fülle des Haares.

Die EP-A-729 742 beschreibt Haarbehandlungsmittel auf Basis A) eines Amino-modifizierten Siliconterpolymers und B) mindestens eines kationischen, siliconfreien Konditionierungsmittels auf Basis eines quaternären Ammoniumsalzes.

Nachteilig an der in den beiden zuvor genannten Publikationen beschriebenen Verwendung von Polysiloxanen, die nicht kovalent an das Festigerpolymer gebunden sind, ist es, dass häufig Entmischungen der Formulierungen während der Lagerung und nach deren Anwendung auf dem Haar auftreten.

Die EP-A-408 311 beschreibt die Verwendung von Copolymeren mit Einheiten aus a) ethylenisch ungesättigten, hydrophilen Monomeren und b) ethylenisch ungesättigten Monomeren mit Polysiloxangruppen in Haarpflegeprodukten.

Die EP-A-412 704 beschreibt ein Haarpflegemittel auf Basis eines Pfropfcopolymers, welches monovalente Siloxan-Polymereinheiten an einem Rückgrat auf Basis eines Vinylpolymers aufweist. Nach dem Trocknen zerfällt das Polymer in eine diskontinuierliche, siliconhaltige Phase und eine kontinuierliche, siliconfreie Phase.

Die WO 93/03703 beschreibt eine Haarsprayzusammensetzung, umfassend: a) 0,1 bis 2 Gew.-% eines oberflächenaktiven Mittels, b) 0,5 bis 15 Gew.-% eines ionischen Harzes mit einem zahlenmittleren Molekulargewicht von mindestens 300 000 und c) einen flüssigen Träger. Dabei umfasst das ionische Harz siliconhaltige Monomere, wobei das Harz nach dem Trocknen in eine diskontinuierliche, siliconhaltige Phase und eine siliconfreie kontinuierliche Phase zerfällt.

Die EP-A-362 860 beschreibt Alkohol-modifizierte Siliconesterderivate und kosmetische Zusammensetzungen, die diese enthalten.

Keine dieser Publikationen beschreibt Festigerpolymere auf Basis von Polyurethanen mit einer kovalenten Bindung der Siloxangruppen an die Festigerpolymere über stickstoffhaltige Gruppen. Nachteilig an den zuvor genannten siloxanhaltigen Polymeren ist zum einen die aufwendige Synthese der zugrunde liegenden Monomeren. Zudem können nicht umgesetzte Makromonomere sowie herstellungsbedingt enthaltene, nicht polymerisierbare Verunreinigungen aufgrund ihres hohen Molekulargewichtes nicht oder nur mit erheblichem Aufwand von den siloxanhaltigen Festigerpolymeren abgetrennt werden. Diese Komponenten sind jedoch im Allgemeinen toxikologisch und allergologisch nicht ganz unbedenklich. Des Weiteren eignen sich diese Polymere nur sehr eingeschränkt zur Herstellung VOC-armer Formulierungen.

Es ist bekannt, in Wasser lösliche oder dispergierbare Polyester, Polyamide oder auch Polyurethane aufgrund ihrer filmbildenden Eigenschaften und einer im Allgemeinen niedrigen Viskosität in Wasser/Ethanol in der Kosmetik einzusetzen. So beschreibt die US-A-4,743,673 hydrophile Polyurethanpolymere mit Carboxygruppen im Polymerrückgrat. Diese Polyurethane sind aufgebaut aus einer Polyolkomponente,bei der es sich um ein Alkylenglykol, ein Polyoxyalkylenglykol, oder ein lineares Polyesterdiol handeln kann, einer Carbonsäureesterkomponente mit Hydroxy- oder Aminogruppen und einem organischen Isocyanat oder Isocyanat-Vorläufer. Das Polyurethan enthält also an das Polymerrückgrat gebundene Estergruppen, die anschließend durch 30- bis 60-minütiges Erhitzen mit einer starken Base, wie Natrium- oder Kaliumhydroxid, unter Rückfluss verseift werden. Das erhaltene Produkt ist sowohl in Wasser als auch in Ethanol nicht mehr klar löslich. Insbesondere bei Verwendung eines Polyesterdiols als Polyolkomponente erfolgt aufgrund der Behandlung mit der starken Base unter Rückflussbedingungen nicht nur eine Verseifung der Estergruppen der Carbonsäureesterkomponente, sondern auch eine Verseifung der in der Polyurethankette enthaltenen Estergruppierungen. Es kommt somit zur Spaltung der Polyurethankette und zu einer drastischen Verringerung des Molekulargewichts der Polyurethane. Eine Anwendung der Polyurethane in Haarsprays ist zwar erwähnt. In der Praxis sind die mit diesen Polyurethanen erhaltenen Filme für die Haarkosmetik aber nicht brauchbar, weil sie entweder wasserunlöslich sind oder ein zu geringes Molekulargewicht und somit unzureichende Festigungswirkung besitzen.

Die DE-A-42 25 045 beschreibt die Verwendung von wasserlöslichen oder in Wasser dispergierbaren, anionischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Sie besitzen eine Glastemperatur von mindestens 15°C und Säurezahlen von 12 bis 150. Polyurethane auf Basis von Polytetrahydrofuranen und/oder Polysiloxanen sind nicht beschrieben. Haarfestigerpolymere auf Basis dieser Polyurethane sind im Hinblick auf die Elastizität verbesserungswürdig und weisen im Allgemeinen keinen angenehmen Griff auf.

Die DE-A-42 41 118 beschreibt die Verwendung von kationischen Polyurethanen und Polyharnstoffen als Hilfsmittel in kosmetischen und pharmazeutischen Zubereitungen. Sie finden insbesondere Anwendung als Filmbildner in Haarfestigern. Sie sind aufgebaut aus
a) mindestens einem Diisocyanat, welches bereits vorher mit einer oder mehreren Verbindungen, die zwei oder mehrere aktive Wasserstoffatome pro Molekül enthalten, umgesetzt sein kann und
b) mindestens einem Diol, primären oder sekundären Aminoalkohol, primären oder sekundären Diamin oder primären oder sekundären Triamin mit einem oder mehreren tertiären, quartären oder protonierten tertiären Aminstickstoffatomen.

Die Polymere besitzen eine Glasübergangstemperatur von mindestens 25°C und eine Aminzahl von 50 bis 200, bezogen auf die nicht-quaternisierten oder protonierten Verbindungen. Polyurethane mit kationischen Gruppen bilden hygroskopische Filme aus, die klebrig sind. Sie erfüllen daher im Allgemeinen nicht die Ansprüche bezüglich Glanz und natürlichem Aussehen, die derzeit an Haarfestigerpolymere gestellt werden.

Die EP-A-619 111 beschreibt die Verwendung von Polyurethanen auf Basis von organischen Diisocyanaten, Diolen und 2,2-Hydroxymethyl-substituierten Carboxylaten der Formel 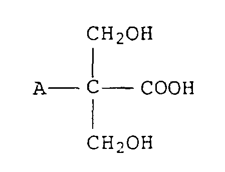 worin A für ein Wasserstoffatom oder eine C₁-C₂₀-Alkylgruppe steht, in Haarfixierungsmitteln. Zumindest ein Teil der Carbonsäuregruppen wird dabei mit einer organischen oder anorganischen Base neutralisiert. Die Diole weisen dabei ein Molekulargewicht im Bereich von 300 bis 20 000 auf, wobei als geeignete Diolkomponente unter anderem auch Polytetrahydrofurane genannt werden. Keines der Ausführungsbeispiele beschreibt jedoch ein Polyurethan auf Basis eines Polytetrahydrofurans. Polyurethane, die ein polymeres Diol, ein Polyesterdiol und eine niedermolekulare Verbindung mit zwei aktiven Wasserstoffatomen einpolymerisiert enthalten, sind nicht beschrieben. Ebenso wenig sind Polyurethane auf Basis von Polysiloxanen beschrieben. Filme auf Basis dieser Polyurethane sind weich und klebrig und die darauf basierenden Haarfestiger entsprechend verbesserungswürdig.

Die in den zuletzt erwähnten Publikationen beschriebenen Polyurethane können die Anforderungen an Haarfestigerpolymere nur teilweise erfüllen. So ist die erwünschte Geschmeidigkeit des Haares bei allen zuvor genannten Produkten auf Polyurethanbasis verbesserungswürdig.

Die EP-A-636 361 beschreibt eine kosmetische Zusammensetzung, welche in einem kosmetisch verträglichen Träger mindestens einen Pseudolatex auf Basis eines Polykondensates umfasst, das mindestens eine Polysiloxaneinheit und mindestens eine Polyurethan- und/oder Polyharnstoffeinheit mit anionischen oder kationischen Gruppen umfasst. Bei den anionischen Gruppen handelt es sich um vollständig oder teilweise neutralisierte Carbonsäure- oder Sulfonsäuregruppen, bei den kationischen Gruppen um vollständig oder teilweise neutralisierte und/oder quaternisierte tertiäre Amine. Polyurethane auf Basis von Polytetrahydrofuranen und/oder Polysiloxanen, Polyesterdiolen und einer niedermolekularen Verbindung mit zwei aktiven Wasserstoffatomen sind nicht beschrieben. Die WO 97/25021 hat einen vergleichbaren Offenbarungsgehalt. Diese kosmetischen Mittel eignen sich unter anderem zur Behandlung von keratinischen Materialien. Die Auswaschbarkeit dieser Filmbildner ist jedoch nicht zufriedenstellend. Zudem besitzen sie aufgrund eines hohen Siloxananteils auch nicht die für ein Haarpolymer erforderliche Festigungswirkung.

Die DE-A-195 41 329 und die WO 97/17052 beschreiben Haarbehandlungsmittel, enthaltend ein in Wasser lösliches oder dispergierbares Salz der Formel I:

[A-(X)ₙ]ⁿ⁻. [HₘB]^{m+} I

worin
- A: für einen kosmetisch akzeptablen aliphatischen, cycloaliphatischen oder aromatischen Rest steht, der siloxan- und/oder fluorhaltige Einheiten aufweisen kann,
- X: für eine Carboxylat-, Sulfonat-, Phosphat- oder Phosphonatgruppe steht;
- B: eine kosmetisch akzeptable Aminbase bedeutet die siloxan- und/oder fluorhaltige Einheiten aufweisen kann;
- n: für 1 bis 30 steht; und
- m: die Wertigkeit des Amins B bedeutet.

Haarspray-Formulierungen auf Basis dieser siloxanhaltiger Salze, einem nicht siloxanhaltigen Haarfestigerpolymer und einem Siliconöl führen zu Filmen, die leicht, z. B. durch mechanische Belastung, von der Haaroberfläche abgelöst werden. Die Festigungswirkung dieser Formulierungen ist daher verbesserungswürdig.

Die DE-A-195 41 326 und die WO 97/17386 beschreiben wasserlösliche oder wasserdispergierbare Polyurethane mit endständigen Säuregruppen, ihre Herstellung und ihre Verwendung. Dabei wird ein in Wasser lösliches oder dispergierbares Polyurethanpräpolymer mit endständigen Isocyanatgruppen mit einer Aminosulfonsäure oder Aminocarbonsäure, insbesondere Taurin, Asparaginsäure und Glutaminsäure, umgesetzt. Haarspray auf Basis dieser Polyurethane sind noch verbesserungswürdig. So müssen die eingesetzten Aminocarboxylate und -sulfonate jeweils frisch aus den entsprechenden Säuren durch Neutralisation hergestellt werden. Insbesondere bei der Formulierung von Haarsprays mit einem hohen Treibgasgehalt und/ oder einem hohen Gehalt an organischen Lösungsmitteln und gegebenenfalls gleichzeitigem Einsatz von Sprühzerstäubern zur Erzielung sehr kleiner Tröpfchen können Probleme auftreten.

Der vorliegenden Erfindung liegt die Aufgabe zugrunde, neue kosmetische Mittel, insbesondere Haarbehandlungsmittel auf Polyurethanbasis, zur Verfügung zu stellen, die einerseits als Haarfestiger brauchbar sind, andererseits aber auch eine gute Auswaschbarkeit (Redispergierbarkeit) besitzen. Sie sollen dem Haar Glätte und Geschmeidigkeit verleihen.

Überraschenderweise wurde nun gefunden, dass diese Aufgabe durch wasserlösliche beziehungsweise durch wasserdispergierbare Polyurethane gelöst wird, welche mindestens ein Polytetrahydrofuran, ein Polysiloxan oder eine Mischung davon und mindestens ein Polyesterdiol enthalten.

Gegenstand der vorliegenden Erfindung ist daher ein wässriges kosmetisches Mittel, enthaltend wenigstens ein wasserlösliches oder wasserdispergierbares Polyurethan aus
a) mindestens einem Polymerisat mit zwei aktiven Wasserstoffatomen pro Molekül, welches ausgewählt ist unter Polytetrahydrofuranen, Polysiloxanen und Mischungen davon,
b) mindestens einem Polyesterdiol,
c) mindestens einer Verbindung mit einem Molekulargewicht im Bereich von 56 bis 300, die zwei aktive Wasserstoffatome pro Molekül enthält,
d) mindestens einer Verbindung, die zwei aktive Wasserstoffatome und mindestens eine anionogene bzw. anionische Gruppe pro Molekül aufweist,
e) mindestens einem Diisocyanat,
oder die Salze davon, wobei das Polyurethan keine von einem primären oder sekundären Amin, welches eine ionogene bzw. ionische Gruppe aufweist, stammende Einheit enthält.

Bei der Komponente a) handelt es sich bevorzugt um ein Polymerisat mit einem zahlenmittleren Molekulargewicht im Bereich von etwa 400 bis 4 000, bevorzugt 500 bis 4 000, insbesondere 600 bis 3 000. Geeignete Polytetrahydrofurane können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Bei den Polysiloxanen a) handelt es sich vorzugsweise um eine Verbindung der Formel I 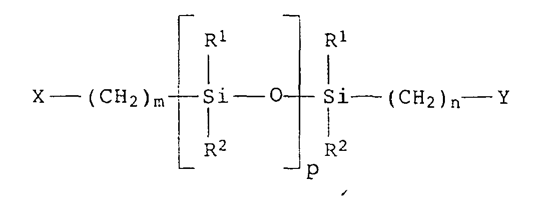 worin
- R¹ und R²: unabhängig voneinander für C₁- bis C₄-Alkyl, Benzyl oder Phenyl stehen,
- X und Y: unabhängig voneinander für OH oder NHR³ stehen, wobei R³ für Wasserstoff, C₁- bis C₆-Alkyl oder C₅- bis C₈-Cycloalkyl steht,
- m und n: unabhängig voneinander für 2 bis 8 stehen,
- p: für 3 bis 50 steht.

Geeignete Alkylreste sind z. B. Methyl, Ethyl, n-Propyl, Isopropyl, n-Butyl, t.-Butyl, n-Pentyl, n-Hexyl etc. Geeignete Cycloalkylreste sind z. B. Cyclopentyl, Cyclohexyl, Cycloheptyl, Cyclooctyl etc.

Vorzugsweise stehen R¹ und R² beide für Methyl.

Brauchbare Polyesterdiole b) weisen ein zahlenmittleres Molekulargewicht im Bereich von etwa 400 bis 5 000, bevorzugt 500 bis 3 000, insbesondere 600 bis 2 000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel 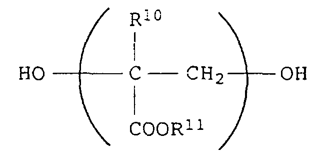 worin R¹⁰ für H oder CH₃ steht und R¹¹ für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer^{®}-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/ Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/ Diethylenglykol/Dimethylolcyclohexan.

Bei der Komponente c) handelt es sich bevorzugt um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein. Die resultierenden Polyurethane sind dabei im Wesentlichen unvernetzt.

Bevorzugt werden als Komponente c) Diole eingesetzt. Brauchbare Diole sind z. B. Ethylenglykol, Propylenglykol, Butylenglykol, Neopentylglykol, Cyclohexandimethylol, Di-, Tri-, Tetra-, Penta-oder Hexaethylenglykol und Mischungen davon. Bevorzugt werden Neopentylglykol und/oder Cyclohexandimethylol eingesetzt.

Geeignete Aminoalkohole sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine sind z. B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Geeignete Verbindungen d), die zwei aktive Wasserstoffatome und mindestens eine anionogene oder anionische Gruppe pro Molekül aufweisen, sind z. B. Verbindungen mit Carboxylat- und/oder Sulfonatgruppen. Als Komponente d) sind Dimethylolpropansäure und Mischungen, die Dimethylolpropansäure enthalten, besonders bevorzugt.

Als Komponente d) brauchbar sind auch Verbindungen der Formeln

H₂N(CH₂)ₙ-NH-(CH₂)ₘ-COO⁻M⁺

H₂N(CH₂)ₙ-NH-(CH₂)ₘ-SO₃⁻ M⁺

worin m und n unabhängig voneinander für eine ganze Zahl von 1 bis 8, insbesondere 1 bis 6, stehen und M für Li, Na oder K steht. Bevorzugt werden als Komponente d) Mischungen eingesetzt, die Dimethylolpropansäure und bis zu 3 Gew.-%, bezogen auf die Gesamtmenge der Komponenten a) bis e), mindestens einer Verbindung der zuvor genannten Formeln aufweisen.

Bei der Komponente e) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Vorzugsweise enthalten die erfindungsgemäßen Mittel wenigstens ein Polyurethan aus
- 0,5 bis 40 Gew.-%, bevorzugt 2 bis 30 Gew.-%, wenigstens einer Komponente a),
- 1 bis 45 Gew.-%, bevorzugt 2 bis 35 Gew.-%, wenigstens eines Polyesterdiols b),
- 0,3 bis 15 Gew.-%, bevorzugt 0,5 bis 12 Gew.-%, wenigstens einer Komponente c),
- 5 bis 25 Gew.%, bevorzugt 8 bis 20 Gew.-%, wenigstens einer Komponente d),
- 25 bis 60 Gew.-%, bevorzugt 35 bis 53 Gew.-%, wenigstens einer Komponente e).

Nach einer bevorzugten Ausführungsform enthalten die erfindungsgemäßen Mittel wenigstens ein Polyurethan, welches als Komponente a) im Wesentlichen oder ausschließlich ein oder mehrere Tetrahydrofurane einpolymerisiert enthält. Bevorzugt handelt es sich dabei dann um ein Polyurethan aus
- 5 bis 40 Gew.-%, bevorzugt 10 bis 35 Gew.-%, wenigstens eines Polytetrahydrofurans a),
- 1 bis 40 Gew.-%, bevorzugt 2 bis 30 Gew.-%, wenigstens eins Polyesterdiols b),
- 0,3 bis 15 Gew.-%, bevorzugt 0,5 bis 12 Gew.-%, wenigstens einer Komponente c),
- 5 bis 25 Gew.-%, bevorzugt 8 bis 20 Gew.-%, wenigstens einer Komponente d),
- 25 bis 60 Gew.-%, bevorzugt 35 bis 53 Gew.-%, wenigstens einer Komponente e),
wobei das Polytetrahydrofuran a) bis zu einem Anteil von etwa 8 Gew.-%, bevorzugt 5 Gew.-%, bezogen auf die Gesamtmenge der Komponenten a) bis e), durch ein Polysiloxan ersetzt sein kann.

Nach einer weiteren bevorzugten Ausführungsform enthalten die erfindungsgemäßen Mittel wenigstens ein Polyurethan, welches als Komponente a) im Wesentlichen oder ausschließlich eine oder mehrere, Polysiloxane einpolymerisiert enthält. Vorzugsweise handelt es sich dabei um ein Polyurethan aus
- 0,2 bis 20 Gew.-%, bevorzugt 0,5 bis 15 Gew.-%, insbesondere 1 bis 10 Gew.-%, wenigstens eines Polysiloxans a),
- 10 bis 45 Gew.-%, bevorzugt 15 bis 40 Gew.-%, wenigstens eines Polyesterdiols b),
- 0,3 bis 15 Gew.-%, bevorzugt 0,5 bis 12 Gew.-%, wenigstens einer Komponente c),
- 5 bis 25 Gew.-%, bevorzugt 8 bis 20 Gew.-%, wenigstens einer Komponente d),
- 25 bis 60 Gew.-%, bevorzugt 35 bis 53 Gew.-%, wenigstens einer Komponente e).

Gewünschtenfalls kann das Polyesterdiol b) teilweise durch eine äquimolare Menge eines Polytetrahydrofurans a) ersetzt sein.

Die Herstellung der in den erfindungsgemäßen Mitteln eingesetzten Polyurethane erfolgt durch Umsetzung der Verbindungen der Komponenten a), b), c) und d) mit der Komponente e). Die Temperatur liegt dabei in einem Bereich von etwa 60 bis 140 °C, bevorzugt etwa 70 bis 100 °C. Die Reaktion kann ohne Lösungsmittel oder in einem geeigneten inerten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Die Komponenten werden in solchen Mengen eingesetzt, dass das Verhältnis von NCO-Äquivalent der Verbindungen der Komponente e) zu Äquivalent aktives Wasserstoffatom der Komponenten a), b), c) und d) in einem Bereich von etwa 0,8:1 bis 1,25:1, bevorzugt 0,85:1 bis 1,2:1, insbesondere 1,05:1 bis 1,15:1, liegt. Weisen die resultierenden Polyurethane noch freie Isocyanatgruppen auf, so werden diese abschließend durch Zusatz von Aminen, vorzugsweise Aminoalkoholen inaktiviert. Geeignete Aminoalkohole sind die zuvor als Komponente c) beschriebenen, bevorzugt 2-Amino-2-methyl-1-propanol.

Die Säuregruppen enthaltenden Polyurethane können durch teilweise oder vollständige Neutralisation mit einer Base in eine wasserlösliche bzw. wasserdispergierbare Form überführt werden.

In aller Regel weisen die erhaltenen Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethanolamine, bevorzugt Methyl- oder Ethyldiethanolamin und Di-C₁-C₆-Alkylethanolamine. Besonders für den Einsatz in Haarbehandlungsmitteln haben sich zur Neutralisation der Säuregruppen enthaltenden Polyurethane 2-Amino-2-methyl-1-propanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 20 bis 40 % oder vollständig, d. h. zu 100 % erfolgen.

Wird bei der Herstellung der Polyurethane ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem Polyurethan zusätzlich Wasser zugegeben werden. Nach Ersatz des Lösungsmittels durch Wasser erhält man eine Lösung oder Dispersion des Polymers, aus der, falls gewünscht, das Polymer in üblicher Weise gewonnen werden kann, z. B. durch Sprühtrocknung.

Die in den erfindungsgemäßen Mitteln eingesetzten Polyurethane weisen K-Werte (gemessen nach E. Fikentscher, Cellulose-Chemie 13 (1932), S. 58-64, an einer 1%igen Lösung in N-Methylpyrrolidon) in einem Bereich von 15 bis 90, bevorzugt 20 bis 60, auf. Ihre Glasübergangstemperatur beträgt im Allgemeinen mindestens 0 °C, bevorzugt mindestens 20 °C, insbesondere bevorzugt mindestens 25 °C und speziell mindestens 30 °C. Enthalten die erfindungsgemäßen Mittel Polyurethane mit von Polysiloxanen der Formel I abgeleiteten Einheiten, so beträgt der auf den Feststoffgehalt der erfindungsgemäßen Polyurethane bezogene Anteil an Siloxangruppen im Allgemeinen etwa 0,05 bis 20 Gew.-%, bevorzugt etwa 0,05 bis 15 Gew.-%, insbesondere 0,05 bis 10 Gew.-%. Mittel, enthaltend wenigstens ein Polyurethan mit einem Siloxangehalt in einem Bereich von etwa 5 bis 20 Gew.-%, bevorzugt 7 bis 17 Gew.-%, eignen sich vorzugsweise als Lösungsvermittler für hydrophobe Produkte, insbesondere Silicone, und als Additive für Haarbehandlungsmittel.

Die erfindungsgemäßen kosmetischen Mittel eignen sich insbesondere als Beschichtungsmittel für keratinhaltige Oberflächen (Haar, Haut und Nägel). Sind die in den erfindungsgemäßen Mitteln eingesetzten Verbindungen wasserdispergierbar, können sie in Form von wässrigen Mikrodispersionen mit Teilchendurchmessern von üblicherweise 1 bis 150 nm, bevorzugt 5 bis 100 nm, zur Anwendung gebracht werden. Die Feststoffgehalte der Präparate liegen dabei üblicherweise in einem Bereich von etwa 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, insbesondere in Form eines Haarsprays vorliegen. Zur Anwendung als Haarfestiger sind dabei Mittel bevorzugt, die Polyurethane enthalten, deren Glasübergangstemperatur T_{g} ≥ 20 °C, bevorzugt ≥ 30 °C, ist. Der K-Wert dieser Polymere liegt vorzugsweise in einem Bereich von 23 bis 90, insbesondere 25 bis 60. Werden in Mitteln zur Anwendung als Haarfestiger Polyurethane eingesetzt, die von Polysiloxanen abgeleitete Einheiten aufweisen, so beträgt der auf den Feststoffgehalt bezogene Anteil an Siloxangruppen im Allgemeinen höchstens 15 Gew.-%.

Im Allgemeinen enthalten die erfindungsgemäßen Mittel die Polyurethane in einer Menge im Bereich von 0,2 bis 20 Gew.-%, bezogen auf das Gesamtgewicht des Mittels.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter enthalten die erfindungsgemäßen Haarbehandlungsmittel im Allgemeinen übliche kosmetische Hilfsstoffe, beispielsweise Weichmacher, wie Glycerin und Glykol; Emollienzien; Parfüms; UV-Absorber; Farbstoffe; Verdickungsmittel; antistatische Mittel; Mittel zur Verbesserung der Kämmbarkeit; Konservierungsmittel; und Schaumstabilisatoren.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen;
- amphotere oder zwitterionische Polymere, wie die unter den Bezeichnungen Amphomer^{®} (Delft National) erhältlichen Octyl-acrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacryloylethylbetain/Methacrylat-Copolymere, die unter der Bezeichnung Amersette^{®} (AMERCHOL) im Handel erhältlich sind;
- anionische Polymere, wie Vinylacetat/Crotonsäure-Copolymere, wie sie beispielsweise unter den Bezeichnungen Resyn^{®} (NATIONAL STARCH), Luviset^{®} (BASF) und Gafset^{®} (GAF) im Handel sind, Vinylpyrrolidon/Vinylacrylat-Copolymere, erhältlich beispielsweise unter dem Warenzeichen Luviflex^{®} (BASF). Ein bevorzugtes Polymer ist das unter der Bezeichnung Luviflex^{®} VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold^{®} strong (BASF) vertrieben werden, sowie Luvimer^{®} (BASF, Terpolymer aus t-Butylacrylat, Ethylacrylat und Methacrylsäure), oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat^{®} (Copolymer aus Vinylpyrrolidon und Vinylimidazoliummethochlorid), Luviquat^{®} Hold (Copolymerisat aus quaternisiertem N-Vinylimidazol, N-Vinylpyrrolidon und N-Vinylcaprolactam), Merquat^{®} (Polymer auf Basis von Dimethyldiallylammoniumchlorid), Gafquat^{®} (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren^{®} (Fa. Goldschmidt) oder Belsil^{®} (Fa. Wacker).

Die zuvor beschriebenen Polyurethane auf Basis von mindestens einem Polytetrahydrofuran und/oder Polysiloxan und mindestens einem Polyesterdiol werden vorzugsweise als Mischung mit einem anderen amidgruppenhaltigen Haarpolymer eingesetzt. Dazu zählen z. B. die in der DE-A-42 25 045 beschriebenen Polyurethane, die zuvor beschriebenen Vinylpyrrolidon/Acrylat-Terpolymere und Acrylsäure/ Ethylacrylat/N-tert.-Butylacrylamid-Terpolymere (z. B. Ultrahold^{®}strong der BASF AG), die zuvor beschriebenen amidgruppenhaltigen amphoteren Polymere (z. B. Amphomer^{®}) und insbesondere Copolymerisate, die einen Anteil an amidgruppenhaltigen Monomeren, wie N-Vinyllactamen, von mindestens 30 Gew.-% aufweisen (z. B. Luviskol^{®}plus und Luviskol^{®}VA37 der BASF AG). Besonders bevorzugt sind Mischungen aus den zuvor beschriebenen siloxangruppenhaltigen, polyesterdiolhaltigen Polyurethanen mit diesen amidgruppenhaltigen Haarpolymeren.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-% mindestens eines, in Wasser löslichen oder dispergierbaren Polyurethans auf Basis von Polytetrahydrofuranen und/oder Polysiloxanen und von Polyesterdiolen,
b) 40 bis 99 Gew.-%, bevorzugt 50 bis 98 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 50 Gew.-% eines Treibmittels, vorzugsweise Dimethylether,
d) 0 bis 15 Gew.-% mindestens eines von a) verschiedenen, in Wasser löslichen oder dispergierbaren Haarpolymers,
e) 0 bis 0,2 Gew.-% mindestens eines wasserunlöslichen Silicons,
f) 0 bis 2 Gew.-% mindestens eines nichtionischen, siloxanhaltigen, in Wasser löslichen oder dispergierbaren Polymers.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 15 Gew.-%, bevorzugt 0,1 bis 10 Gew.-%, bezogen auf das Gesamtgewicht des Mittels. Bevorzugt können dabei wasserlösliche oder wasserdispergierbare Polyurethane eingesetzt werden, die keine Siloxangruppen einpolymerisiert enthalten.

Das erfindungsgemäße Mittel kann als Komponente e) mindestens ein wasserunlösliches Silicon, insbesondere ein Polydimethylsiloxan, z. B. die Abil^{®}-Typen der Fa. Goldschmidt, enthalten. Der Anteil dieser Komponente beträgt dann im Allgemeinen etwa 0,001 bis 0,2 Gew.-%, bevorzugt 0,01 bis 0,1 Gew.-%, bezogen auf das Gesamtgewicht des Mittels.

Das erfindungsgemäße Mittel kann als Komponente f) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Ein besonders bevorzugtes Haarbehandlungsmittel enthält:
a) 0,5 bis 20 Gew.-% mindestens eines, in Wasser löslichen oder dispergierbaren Polyurethans auf Basis von Polysiloxanen,
b) 50 bis 98 Gew.-% eines Lösungsmittels, ausgewählt unter Wasser, Ethanol und Mischungen davon,
c) 0 bis 50 Gew.-% eines Treibmittels,
d) 0,1 bis 10 Gew.-% mindestens eines in Wasser löslichen oder dispergierbaren, amidgruppenhaltigen, siliconfreien Haarpolymers,
e) 0 bis 0,1 Gew.-% mindestens eines wasserunlöslichen Silicons,
f) 0 bis 1 Gew.-% mindestens eines nichtionischen, siloxanhaltigen, in Wasser löslichen oder dispergierbaren Polymers,
sowie übliche Zusatzstoffe.

Nach einer bevorzugten Ausführungsform handelt es sich bei dem amidgruppenhaltigen Haarpolymer d) um ein Polymerisat, welches ein oder mehrere amidgruppenhaltige Monomere einpolymerisiert enthält. Bevorzugte amidgruppenhaltige Monomere sind N-Vinyllactame, die bevorzugt ausgewählt sind unter N-Vinylpyrrolidon, N-Vinylcaprolactam, deren Derivaten, die z. B. einen oder mehrere C₁- bis C₄-Alkylsubstituenten aufweisen können, und Mischungen davon. Die Haarpolymere d) enthalten diese dann vorzugsweise in einer Menge von mindestens 30 Gew.-% einpolymerisiert. Geeignet ist auch eine Polymermischung, die mindestens ein solches Copolymerisat aufweist. Besonders bevorzugt sind die Luviskol^{®}-Marken der BASF AG, wie Luviskol VA37 und Luviskol plus.

Nach einer weiteren bevorzugten Ausführungsform handelt es sich bei dem amidgruppenhaltigen Haarpolymer d) um ein siliconfreies Polyurethan, wie sie z. B. in der DE-A-42 25 045, DE-A-42 41 118 und EP-A-619 111 beschrieben werden.

Die erfindungsgemäßen Mittel besitzen den Vorteil, dass sie einerseits den Haaren die gewünschte Festigkeit verleihen und andererseits die Polymere leicht auswaschbar (redispergierbar) sind, und sie dem Haar zusätzlich Glätte und/oder Glanz verleihen. Darüber hinaus lassen sich Haarbehandlungsmittel mit einem in VOC-Gehalt von weniger als 85 Gew.-%, bevorzugt weniger als 60 Gew.-%, und auch rein wässrige Formulierungen herstellen, selbst wenn sie als Haarspray formuliert sind.

Die zuvor beschriebenen Polyurethane auf Basis wenigstens eines Polytetrahydrofurans und/oder Polysiloxans eignen sich auch als Hilfsmittel in der Pharmazie, wie z. B. als Überzugsmittel und/ oder Bindemittel für feste Arzneiformen. Sie können auch in Cremes und als Tablettenüberzugsmittel und Tablettenbindemittel eingesetzt werden. Des Weiteren eignen sie sich für die Verwendung als Beschichtungsmittel für die Textil-, Papier-, Druck-, Leder- und Klebstoffindustrie.

Die Erfindung wird anhand der folgenden, nicht einschränkenden Beispiele näher erläutert.

### Beispiele

### Vergleichsbeispiel 1, Beispiele 2 bis 9

### Polyurethanherstellung

In einem Rührapparat, der mit Rührer, Tropftrichter, Thermometer, Rückflusskühler und einer Vorrichtung für das Arbeiten unter Stickstoff ausgestattet war, wurden ein Polyesterdiol (Mₙ = 1 000 g/Mol, hergestellt aus Isophthalsäure, Adipinsäure und Hexandiol), gegebenenfalls ein Polytetrahydrofuran (Mₙ = 1000 g/Mol) (Beispiele 5 bis 9), Neopentylglykol und Dimethylolpropansäure in einer Menge nach Tabelle 1 in Methylethylketon (Feststoffgehalt der resultierenden Reaktionslösung ca. 75 %) unter Erhitzen auf eine Temperatur von 70 °C und unter Rühren gelöst. Anschließend wurde unter Rühren Isophorondiisocyanat in einer Menge nach Tabelle 1 zugetropft, wobei die Reaktionstemperatur anstieg. Bei einer Innentemperatur von 85 °C wurde das Reaktionsgemisch dann solange gerührt, bis der Isocyanatgruppengehalt des Gemisches praktisch konstant blieb (ca. 0,5 bis 0,8 %). Das Reaktionsgemisch wurde mit Aceton auf 40 Gew.-% verdünnt und unter Rühren auf Raumtemperatur abgekühlt. Bei einer Temperatur von etwa 30 °C gab man dann gegebenenfalls ein Polysiloxandiamin (Mₙ = 900 g/Mol, Tegomer^{®} A-Si 2122 der Fa. Goldschmidt, in Form einer 80%igen Lösung in Methylethylketon) (Beispiele 2 bis 4, 6, 7 und 9) in einer Menge nach Tabelle 1 zu dem wie zuvor beschrieben hergestellten Polyurethanpräpolymer. Die Mischung wurde weitere 30 min umgesetzt und anschließend die restlichen Isocyanatgruppen durch Zugabe von 2-Amino-2-methylpropanol inaktiviert. Das Reaktionsgemisch wurde dazu bei einer Temperatur von etwa 40 °C solange gerührt, bis der Isocyanatgruppengehalt des Gemisches im Wesentlichen bei 0 lag. Anschließend gab man Wasser zum Reaktionsgemisch und neutralisierte das Reaktionsprodukt mit 2-Amino-2-methylpropanol (pH etwa 8,0). Das Methylethylketon wurde dann im Vakuum bei 40 °C abdestilliert, wobei eine wässrige Dispersion des Polyurethans erhalten wurde.

Ein pulverförmiges Produkt kann durch Sprühtrocknung erhalten werden.

**Tabelle 1:**

| Bsp. Nr. | Polyester-diol¹⁾ [mol] | Poly (THF)²⁾ [mol] | Poly-siloxan-diamin³⁾ [mol] | NPG⁴⁾ [mol] | DMPA⁵⁾ [mol] | IPDI⁶⁾ [mol] | Siloxan-gehalt [Gew.-%] |
|---|---|---|---|---|---|---|---|
| V1 | 0,8 | - | - | 1,6 | 3,2 | 6,0 | 0 |
| 2 | 0,7 | - | 0,1 | 1,6 | 3,2 | 6,0 | 3,3 |
| 3 | 0,6 | - | 0,2 | 1,6 | 3,2 | 6,0 | 6,65 |
| 4 | 0,3 | - | 0,5 | 1,6 | 3,2 | 6,0 | 16,8 |
| 5 | 0,4 | 0,4 | - | 1,6 | 3,2 | 6,0 | 0 |
| 6 | 0,4 | 0,35 | 0,05 | 1,6 | 3,2 | 6,0 | 1,65 |
| 7 | 0,4 | 0,3 | 0,1 | 1,6 | 3,2 | 6,0 | 3,3 |
| 8 | 0,2 | 0,6 | - | 1,6 | 3,2 | 6,0 | 0 |
| 9 | 0,2 | 0,55 | 0,05 | 1,6 | 3,2 | 6,0 | 1,65 |

| | | | | | | | |
|---|---|---|---|---|---|---|---|
| ¹⁾ Polyesterdiol aus Isophthalsäure, Adipinsäure, Hexandiol, Mₙ = 1 000 g/mol ²⁾ Polytetrahydrofuran, Mₙ = 1 000 g/mol ³⁾ Polysiloxandiamin, Mₙ = 900 g/mol ⁴⁾ NPG = Neopentylglykol ⁵⁾ DMPA = Dimethylolpropansäure ⁶⁾ IPDI = Isophorondiisocyanat | | | | | | | |

### Anwendungstechnische Beispiele

### Beispiele 10 bis 18

Aerosol-Haarspray-Formulierungen mit einem VOC-Gehalt von 97 Gew.-%:

| | | |
|---|---|---|
| Polyurethan gemäß Beispiel 1-9 | 3,00 | Gew.-% |
| Ethanol | 62,00 | Gew.-% |
| Dimethylether | 34,96 | Gew.-% |
| Parfüm, Zusatzstoffe | q.s. | |

### Beispiele 19 bis 27

Aerosol-Haarspray-Formulierungen mit einem VOC-Gehalt von 97 Gew.-%:

| | | |
|---|---|---|
| Polyurethan gemäß Beispiel 1-9 Siloxanfreies, amidgruppenhaltiges, Haarpolymer (Luviskol^{®}Plus der | 1,50 | Gew.-% |
| Fa. BASF AG) | 1,50 | Gew.-% |
| Ethanol | 62,00 | Gew.-% |
| Dimethylether | 34,96 | Gew.-% |
| Parfüm, Zusatzstoffe | q.s. | |

### Beispiele 28 bis 36

Kompakte Aerosol-Haarspray-Formulierungen mit einem VOC-Gehalt von 90 Gew.-%:

| | | |
|---|---|---|
| Polyurethan gemäß Beispiel 1-9 | 10,00 | Gew.-% |
| Ethanol | 55,00 | Gew.-% |
| Dimethylether | 34,96 | Gew.-% |
| Parfüm, Zusatzstoffe | q.s. | |

### Beispiele 37 bis 45

Haarspray-Formulierungen mit einem VOC-Gehalt von 80 Gew.-%:

| | | |
|---|---|---|
| Polyurethan gemäß Beispiel 1-9 | 5,00 | Gew.-% |
| Ethanol | 45,00 | Gew.-% |
| Wasser | 15,00 | Gew.-% |
| Dimethylether | 34,96 | Gew.-% |
| Parfüm, Zusatzstoffe | q.s. | |

### Beispiele 46 bis 54

Haarspray-Formulierungen mit einem VOC-Gehalt von 80 Gew.-%:

| | | |
|---|---|---|
| Polyurethan gemäß Beispiel 1-9 Siloxanfreies, amidgruppenhaltiges, Haarpolymer (Luviskol^{®}Plus der | 2,50 | Gew.-% |
| Fa. BASF AG) | 2,50 | Gew.-% |
| Wasser | 15,00 | Gew.-% |
| Dimethylether | 34,96 | Gew.-% |
| Parfüm, Zusatzstoffe | q.s. | |

### Beispiele 55 bis 63

Haarspray-Formulierungen mit einem VOC-Gehalt von 55 Gew.-%:

| | | |
|---|---|---|
| Polyurethan gemäß Beispiel 1-9 | 5,00 | Gew.-% |
| Ethanol | 20,00 | Gew.-% |
| Wasser | 40,00 | Gew.-% |
| Dimethylether | 34,96 | Gew.-% |
| Parfüm, Zusatzstoffe | q.s. | |

### Beispiele 64 bis 72

Pump-Haarspray-Formulierungen mit VOC-Gehalt 0:

| | | |
|---|---|---|
| Polyurethan gemäß Beispiel 1-9 | 10,00 | Gew.-% |
| Wasser | 89,97 | Gew.-% |
| Parfüm, Zusatzstoffe | q.s. | |

### Filmbewertung:

Die zuvor genannten Haarspray-Formulierungen lieferten klare, feste Filme, die sich hinsichtlich ihrer Flexibilität und Glätte unterscheiden. Die Formulierungen auf Basis von Polyurethanen nach Vergleichsbeispiel 1 sind wenig flexibel und die resultierenden Filme sind nicht glatt. Die Formulierungen auf Basis von Polyurethanen nach Beispiel 4 mit einem hohen Siloxananteil liefern sehr weiche und leicht klebrige Filme. Diese Polyurethane sind alleine nicht als Haarfestigerpolymer geeignet, sie können jedoch als Additiv mit einem anderen Festigerpolymer zu siliconhaltigen Haarsprays mit sehr guten Eigenschaften formuliert werden.

Die Polyurethane aus Vergleichsbeispiel 1 und den Beispielen 2 bis 9 wurden einzeln oder in Form von Mischungen sowie gegebenenfalls unter Zusatz eines wasserunlöslichen Silicons zu einer 5 gew.-%igen ethanolischen Lösung formuliert. Die Zusammensetzungen dieser Formulierungen sind in Tabelle 3 wiedergegeben. Sie wurden auf eine Glasplatte aufgetragen und die resultierenden Filme wurden im Hinblick auf 4 Kriterien, die in Tabelle 2 angegeben sind, geprüft und mit Noten von 1 bis 4 bewertet. Die Bewertungen der Filme sind ebenfalls in Tabelle 3 wiedergegeben.

**Tabelle 2: Bewertungskriterien**

| | | Note |
|---|---|---|
| A) Elastizität/Klebrigkeit | spröde (leicht klebrig) | 4 |
| | weich (leicht klebrig) | 3 |
| | hart (nichtklebrig) | 2 |
| | elastisch (nichtklebrig) | 1 |
| B) Glätte | bremsend | 4 |
| | mäßig-glatt | 3 |
| | glatt | 2 |
| | sehr glatt | 1 |
| C) Auswaschbarkeit | schlecht | 4 |
| | mäßig | 3 |
| | gut | 2 |
| | sehr gut | 1 |
| D) Aussehen | ungleichmäßig trüb | 4 |
| | ungleichmäßig klar | 3 |
| | gleichmäßig klar | 2 |
| | gleichmäßig-glänzend | 1 |

**Tabelle 3: Filmeigenschaften der Polyurethane**

| Polymerlösung¹⁾ | Polyurethan aus Herstellungsbeispiel Nr. (Gew.-%) | Polydimethylsiloxan²⁾ [Gew.-%] | Bewertung | | | |
|---|---|---|---|---|---|---|
| | | | A | B | C | D |
| VL1 | 1 (5) | - | 2 | 2-3 | 3 | 2 |
| VL2 | 1 (5) | 0,0005 | 2 | 2 | 3-4 | 3 |
| L3 | 2 (5) | - | 2 | 1-2 | 3 | 1-2 |
| L4 | 2 (5) | 0,0005 | 2 | 1 | 3 | 1-2 |
| L5 | 3 (5) | - | 1-2 | 1 | 3-4 | 1 |
| L6 | 4 (5) | - | 3 | 1-2 | 2 | 1 |
| L7 | 5 (5) | - | 1 | 2 | 2 | 2 |
| L8 | 6 (5) | - | 1 | 1-2 | 2 | 1 |
| L9 | 6 (5) | 0,0005 | 1 | 1 | 2 | 1 |
| L10 | 8 (5) | - | 1 | 2 | 1-2 | 2 |
| L11 | 1 (3,5)/4 (1,5) | 0,0005 | 2 | 1 | 2-3 | 1 |
| L12 | 7 (3,0)/1 (2,0) | 0,0005 | 1-2 | 1 | 2 | 1 |
| L13 | 4 (4,5)/PVCap³⁾ (0,5) | - | 3 | 1 | 1 | 2 |
| L14 | 4 (2,5)/PVCap (2,5) | - | 1 | 1-2 | 1 | 4 |
| L15 | 4 (0,5)/PVCap (4,5) | - | 2 | 1-2 | 1 | 2 |
| L16 | 3 (2,5)/PVCap (2,5) | - | 1 | 2 | 1 | 1 |
| L17 | 7 (2,5)/PVCap (2,5) | - | 1 | 1-2 | 1 | 1 |

| | | | | | | |
|---|---|---|---|---|---|---|
| ¹⁾ 5 gew.-%ige Lösung in Ethanol ²⁾ Abil^{®}200 der Fa. Goldschmidt ³⁾ PVCap = Polyvinylcaprolactam | | | | | | |

## Patentansprüche

1. Kosmetisches Mittel, enthaltend wenigstens ein wasserlösliches oder wasserdispergierbares Polyurethan aus
a) mindestens einem Polymerisat mit zwei aktiven Wasserstoffatomen pro Molekül, welches ausgewählt ist unter Polytetrahydrofuranen, Polysiloxanen und Mischungen davon,
b) mindestens einem Polyesterdiol,
c) mindestens einer Verbindung mit einem Molekulargewicht im Bereich von 56 bis 300, die zwei aktive Wasserstoffatome pro Molekül enthält,
d) mindestens einer Verbindung, die zwei aktive Wasserstoffatome und mindestens eine anionogene bzw. anionische Gruppe pro Molekül aufweist,
e) mindestens einem Diisocyanat,
oder die Salze davon, wobei das Polyurethan keine von einem primären oder sekundären Amin, welches eine ionogene bzw. ionische Gruppe aufweist, stammende Einheit enthält.

2. Mittel nach Anspruch 1, wobei es sich bei dem Polysiloxan a) um eine Verbindung der Formel I 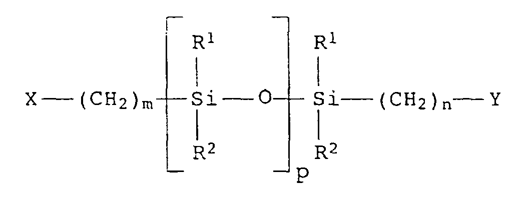 handelt, worin
R¹ und R² unabhängig voneinander für C₁- bis C₄-Alkyl, Benzyl oder Phenyl stehen,
X und Y unabhängig voneinander für OH oder NHR³ stehen, wobei R³ für Wasserstoff, C₁- bis C₆-Alkyl oder C₅- bis C₈-Cycloalkyl steht,
m und n unabhängig voneinander für 2 bis 8 stehen,
p für 3 bis 50 steht.

3. Mittel nach einem der Ansprüche 1 oder 2, wobei es sich bei der Komponente b) um ein Polyesterdiol auf Basis einer aromatischen und einer aliphatischen Dicarbonsäure und eines aliphatischen Diols handelt.

4. Mittel nach einem der vorhergehenden Ansprüche, wobei das Verhältnis von NCO-Äquivalent der Verbindungen der Komponente e) zu Äquivalent aktives Wasserstoffatom der Komponenten a), b), c) und d) in einem Bereich von 0,80:1 bis 1,25:1 liegt.

5. Mittel nach einem der vorhergehenden Ansprüche, wobei freie Isocyanatgruppen des Polyurethans durch Umsetzung mit mindestens einem Aminoalkohol inaktiviert werden.

6. Mittel nach einem der vorhergehenden Ansprüche, wobei anionogene Gruppen der Komponente d) durch Umsetzung mit mindestens einem Aminoalkohol teilweise oder vollständig neutralisiert werden.

7. Mittel nach einem der vorhergehenden Ansprüche, enthaltend ein Polyurethan aus
- 0,5 bis 40 Gew.-% wenigstens einer Komponente a),
- 1 bis 45 Gew.-% wenigstens eines Polyesterdiols b)
- 0,3 bis 15 Gew.-% wenigstens einer Komponente c),
- 5 bis 25 Gew.% wenigstens einer Komponente d)
- 25 bis 60 Gew.-% wenigstens einer Komponente e).

8. Mittel nach einem der vorhergehenden Ansprüche, enthaltend wenigstens ein Polyurethan mit einem Siloxangehalt im Bereich von 5 bis 20 Gew.-% als Lösungsvermittler für hydrophobe Produkte, insbesondere Silicone, und als Additive für Haarbehandlungsmittel.

9. Mittel nach einem der Ansprüche 1 bis 8 in Form eines Haarbehandlungsmittels, insbesondere in Form eines Haarsprays.

10. Mittel nach Anspruch 9, enthaltend
a) 0,5 bis 20 Gew.-% mindestens eines in Wasser löslichen oder dispergierbaren Polyurethans, wie in einem der Ansprüche 1 bis 8 definiert,
b) 40 bis 99 Gew.-% wenigstens eines Lösungsmittels, ausgewählt unter Wasser, wassermischbaren Lösungsmittel und Mischungen davon,
c) 0 bis 50 Gew.-% eines Treibmittels,
d) 0 bis 15 Gew.-% mindestens eines von a) verschiedenen, in Wasser löslichen oder dispergierbaren Haarpolymers,
e) 0 bis 0,2 Gew.-% mindestens eines wasserunlöslichen Silicons,
f) 0 bis 2 Gew.-% mindestens eines nichtionischen, siloxanhaltigen, in Wasser löslichen oder dispergierbaren Polymers.

11. Mittel nach Anspruch 10, wobei die Komponente d) mindestens ein Polymer umfasst, welches wenigstens ein amidgruppenhaltiges Monomer in einer Menge von mindestens 30 Gew.-% einpolymerisiert enthält.

12. Mittel nach Anspruch 10, wobei die Komponente d) mindestens ein Polyurethan umfasst.

13. Verwendung der Polyurethane, wie in einem der Ansprüche 1 bis 8 definiert, als Hilfsmittel in der Kosmetik, insbesondere in der Haarkosmetik, in der Pharmazie, insbesondere in Beschichtungsmitteln oder Bindemitteln für feste Arzneiformen, sowie in Beschichtungsmittel für die Textil-, Papier-, Druck-, Leder- und Klebstoffindustrie.

## Claims

1. A cosmetic composition comprising at least one water-soluble or water-dispersible polyurethane of
a) at least one polymer having two active hydrogen atoms per molecule which is selected from polytetrahydrofurans, polysiloxanes and mixtures thereof,
b) at least one polyesterdiol,
c) at least one compound having a molecular weight in the range from 56 to 300 which contains two active hydrogen atoms per molecule,
d) at least one compound which contains two active hydrogen atoms and at least one anionogenic or anionic group per molecule,
e) at least one diisocyanate,
or a salt thereof, said polyurethane not comprising any unit which originates from a primary or secondary amine having an ionogenic or ionic group.

2. A composition as claimed in claim 1, where the polysiloxane
a) is a compound of the formula I 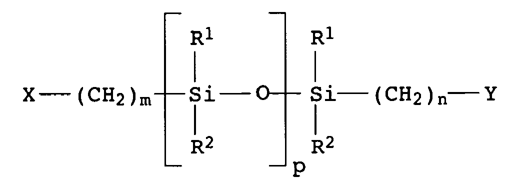
where
R¹ and R² independently of one another are C₁- to C₄-alkyl, benzyl or phenyl,
X and Y independently of one another are OH or NHR³, where R³ is hydrogen, C₁- to C₆-alkyl or C₅- to C₈-cycloalkyl,
m and n independently of one another are from 2 to 8, and
p is from 3 to 50.

3. A composition as claimed in either of claims 1 and 2, where component b) is a polyesterdiol based on an aromatic and an aliphatic dicarboxylic acid and on an aliphatic diol.

4. A composition as claimed in any of the preceding claims, where the ratio of NCO equivalent of the compounds of component e) to equivalent of active hydrogen atom of components a), b), c) and d) is within a range from 0.80:1 to 1.25:1.

5. A composition as claimed in any of the preceding claims, where free isocyanate groups of the polyurethane are deactivated by reaction with at least one amino alcohol.

6. A composition as claimed in any of the preceding claims, where anionogenic groups of component d) are fully or partly neutralized by reaction with at least one amino alcohol.

7. A composition as claimed in any of the preceding claims, comprising a polyurethane of
- from 0.5 to 40% by weight of at least one component a),
- from 1 to 45% by weight of at least one polyesterdiol b),
- from 0.3 to 15% by weight of at least one component c),
- from 5 to 25% by weight of at least one component d),
- from 25 to 60% by weight of at least one component e).

8. A composition as claimed in any of the preceding claims, comprising at least one polyurethane having a siloxane content in the range from 5 to 20% by weight as a solubilizer for hydrophobic products, especially silicones, or as an additive for hair treatment compositions.

9. A composition as claimed in any of claims 1 to 8 in the form of a hair treatment composition, especially in the form of a hairspray.

10. A composition as claimed in claim 9 comprising
a) from 0.5 to 20% by weight of at least one polyurethane which is dispersible or soluble in water, as defined in any of claims 1 to 8,
b) from 40 to 99% by weight of at least one solvent selected from water, water-miscible solvents and mixtures thereof,
c) from 0 to 50% by weight of a propellant,
d) from 0 to 15% by weight of at least one hair polymer which is different from a) and is dispersible or soluble in water,
e) from 0 to 0.2% by weight of at least one water-insoluble silicone,
f) from 0 to 2% by weight of at least one nonionic, siloxane-containing polymer which is dispersible or soluble in water.

11. A composition as claimed in claim 10, where component d) comprises at least one polymer which comprises in copolymerized form at least one amido-containing monomer, in an amount of at least 30% by weight.

12. A composition as claimed in claim 10, where component d) comprises at least one polyurethane.

13. The use of a polyurethane as defined in any of claims 1 to 8 as an auxiliary in cosmetics, especially in hair cosmetics, in pharmacy, particularly in coating compositions or binders for solid drug forms, or in coatings for the textile, paper, printing, leather or adhesives industry.

## Revendications

1. Produit cosmétique contenant au moins un polyuréthanne soluble dans l'eau ou dispersible dans l'eau à base
a) d'au moins un polymère comportant deux atomes d'hydrogène actif par molécule, qui est choisi parmi des polytétrahydrofuranes, des polysiloxanes et leurs mélanges,
b) d'au moins un polyesterdiol,
c) d'au moins un composé présentant un poids moléculaire de l'ordre de 56 à 300, qui contient deux atomes d'hydrogène actif par molécule,
d) d'au moins un composé qui présente deux atomes d'hydrogène actif et au moins un groupe anionogène ou anionique par molécule,
e) d'au moins un diisocyanate,
ou les sels de celui-ci, le polyuréthanne ne contenant aucune unité originaire d'une amine primaire ou secondaire qui présente un groupe ionogène ou ionique.

2. Produit suivant la revendication 1, dans lequel, pour ce qui concerne le polysiloxane a), il s'agit d'un composé de la formule I : 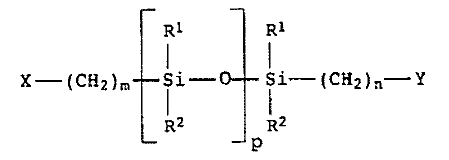 dans laquelle
R¹ et R² représentent indépendamment l'un de l'autre un groupe alkyle en C₁-C₄, benzyle ou phényle,
X et Y représentent indépendamment l'un de l'autre OH ou NHR³, R³ représentant de l'hydrogène ou un groupe alkyle en C₁-C₆ ou cycloalkyle en C₅-C₈,
m et n valant indépendamment l'un de l'autre 2 à 8,
p valant 3 à 50.

3. Produit suivant l'une des revendications 1 et 2, dans lequel, pour ce qui concerne le composant b), il s'agit d'un polyesterdiol à base d'un acide dicarboxylique aromatique et d'un acide dicarboxylique aliphatique et d'un diol aliphatique.

4. Produit suivant l'une des revendications précédentes, dans lequel le rapport entre l'équivalent NCO des composés du composant e) et l'équivalent d'atomes d'hydrogène actif des composants a), b) c) et d) est de l'ordre de 0,80/1 à 1,25/1.

5. Produit suivant l'une des revendications précédentes, dans lequel des groupes isocyanate libres du polyuréthanne sont inactivés par réaction avec au moins un aminoalcool.

6. Produit suivant l'une des revendications précédentes, dans lequel des groupes anionogènes du composant d) sont neutralisés partiellement ou totalement par réaction avec au moins un aminoalcool.

7. Produit suivant l'une des revendications précédentes, contenant un polyuréthanne à base
- de 0,5 à 40% en poids d'au moins un composant a),
- de 1 à 45% en poids d'au moins un polyesterdiol b),
- de 0,3 à 15% en poids d'au moins un composant c),
- de 5 à 25% en poids d'au moins un composant d),
- de 25 à 60% en poids d'au moins un composant e).

8. Produit suivant l'une des revendications précédentes, contenant au moins un polyuréthanne ayant une teneur en siloxane de l'ordre de 5 à 20% en poids comme tiers-solvants pour des produits hydrophobes, en particulier des silicones, et comme additifs pour un produit de traitement de cheveux.

9. Produit suivant l'une des revendications 1 à 8, sous la forme d'un produit de traitement des cheveux, en particulier sous la forme d'une laque pour cheveux.

10. Produit suivant la revendication 9, contenant
a) 0,5 à 20% en poids d'au moins un polyuréthanne soluble ou dispersible dans l'eau, tel que défini dans une des revendications 1 à 8,
b) 40 à 99% en poids d'au moins un solvant, choisi parmi de l'eau, un solvant miscible à l'eau et leurs mélanges,
c) 0 à 50% en poids d'un agent propulseur,
d) 0 à 15% en poids d'au moins un polymère pour les cheveux soluble ou dispersible dans l'eau qui est différent de a),
e) 0 à 0,2% en poids d'au moins une silicone insoluble dans l'eau,
f) 0 à 2% en poids d'au moins un polymère non ionique, contenant du siloxane, soluble ou dispersible dans l'eau.

11. Produit suivant la revendication 10, dans lequel le composant d) comporte au moins un polymère qui contient au moins un monomère contenant des groupes amide en une quantité d'au moins 30% en poids, à l'état copolymérisé.

12. Produit suivant la revendication 10, dans lequel le composant d) comporte au moins un polyuréthanne.

13. Utilisation des polyuréthannes tels que définis dans une des revendications 1 à 8, comme adjuvants en cosmétique, en particulier dans la cosmétique des cheveux, dans la pharmacie, en particulier dans des produits de recouvrement ou des liants pour des formes médicamenteuses solides, ainsi que dans des produits de recouvrement pour l'industrie textile, du papier, de l'impression, du cuir et des colles.
